# FASCICULE DE BREVET EUROPEEN

(11) **EP 0 819 428 B1**
(45) Date de publication et mention de la délivrance du brevet: **09.08.2000**
(21) Numéro de dépôt: 97401458.1
(22) Date de dépôt: 23.06.1997
(51) Int. Cl.: A61K 7/48, A61K 7/027

(54) **Composition comprenant l'association d'un composé volatil et d'un polymère dérivé de polyvinyl-pyrrolidone, et utilisation de ladite association**
Zusammensetzung enthaltend die Kombination einer flüchtige Substanze und einer Polyvinylpyrrolidon-Derivat-Polymer und ihre Verwendung
Composition containing a combination of a volatile compound and a polyvinylpyrrolidone derivative polymer and use thereof

(30) Priorité: 17.07.1996 FR 9608955
(43) Date de publication de la demande: 21.01.1998
(73) Titulaire: L'OREAL, 75008 Paris (FR)
(72) Inventeur: Arnaud, Pascal, 94000 Creteil (FR); Agostini, Isabelle, 92290 Chatenay Malabry (FR)
(74) Mandataire: Dodin, Catherine

(56) Documents cités:
- EP-A- 0 430 473
- EP-A- 0 602 905
- WO-A-96/36308
- WO-A-96/40044
- US-A- 5 389 363
- US-A- 5 480 632
- "Sheer Leg Make-up" HOUSEHOLD & PERSONAL PRODUCTS INDUSTRY, vol. 28, no. 7, juillet 1991, page 18 XP002030730

## Description

La présente invention a pour objet une composition notamment cosmétique pouvant se présenter sous forme d'un stick ou d'une pâte souple, et susceptible d'être utilisée pour le soin et/ou le maquillage de la peau, des semi-muqueuses et/ou des muqueuses, et en particulier des lèvres du visage.

Les compositions cosmétiques ou pharmaceutiques telles que les rouges à lèvres et les fonds de teint, comprennent généralement des corps gras tels que des huiles, des composés pâteux et des cires, ainsi qu'une phase particulaire généralement composée de charges et de pigments.
Ces compositions, lorsqu'elles sont appliquées sur la peau, les muqueuses ou les semi-muqueuses, présentent l'inconvénient de transférer. On entend par là que la composition est susceptible de se déposer, au moins en partie, sur certains supports avec lesquels elle est mise en contact, tels que, par exemple, un verre, une tasse, un vêtement ou la peau.
En se déposant, ladite composition laisse une trace sur ledit support. Il s'en suit donc une persistance médiocre de la composition sur la peau ou les muqueuses, nécessitant de renouveler régulièrement son application. Par ailleurs, l'apparition de traces inacceptables sur certains vêtements et notamment sur les cols de chemisier peut écarter certaines femmes de l'utilisation de ce type de maquillage.
Un autre inconvénient de ces compositions réside dans le problème de migration. On a en effet constaté que certaines compositions avaient tendance à se propager à l'intérieur des ridules et/ou des rides de la peau, dans le cas des fonds de teint; dans les ridules qui entourent les lèvres, dans le cas des rouges à lèvres; dans les plis de la paupière, dans le cas des fards à paupières. On a également constaté, dans le cas notamment des fards à paupières, l'apparition de stries dans le maquillage, générées par les mouvements des paupières.
Tous ces phénomènes engendrent un effet inesthétique que l'on souhaite bien évidemment éviter.
Depuis plusieurs années, de nombreux cosméticiens se sont intéressés aux compositions cosmétiques, notamment de rouge à lèvres ou de fond de teint 'sans transfert'. Ainsi, il a été envisagé, dans la demande de brevet JP-A-61-65809, des compositions de rouge à lèvres 'sans transfert' contenant de 1 à 70% en poids de résine liquide de silicone à motifs répétitifs silicates (ou à réseau tridimensionnel) comportant des chaînes pendantes alkylées de 1 à 6 atomes de carbone ou phénylées, de 10 à 98% en poids d'une huile de silicone volatile à chaîne SI-O cyclique et à radicaux méthyle et des charges pulvérulentes. Ces compositions présentent toutefois l'inconvénient d'être liquides et donc peu commodes à utiliser, ou tout au moins loin du concept classique d'un rouge à lèvres en bâton, limitant ainsi le nombre de femmes susceptibles d'utiliser ce type de rouge à lèvres. De plus, le film obtenu sur les lèvres après évaporation de l'huile de silicone présente l'inconvénient de devenir inconfortable au cours du temps (sensation de dessèchement et de tiraillement).

Plus récemment, il a été envisagé dans la demande de brevet EP-A-602905 des rouges à lèvres 'sans transfert' contenant une silicone volatile cyclique ou linéaire et à chaînes méthylées pendantes et une résine de silicone comportant une chaîne estérifiée pendante ayant au moins 12 atomes de carbone. Le film de rouge à lèvres présente notamment l'inconvénient de manquer de confort à l'application, en particulier d'être trop sec.

D'une manière générale, on sait maintenant que, si l'association d'huiles volatiles avec certains composés siliconés permet d'obtenir un résultat 'sans transfert' satisfaisant, elle présente néanmoins l'inconvénient de conduire, après évaporation des volatils, à un film dont le confort n'est pas optimal, notamment parce qu'il n'est pas possible d'ajouter d'huiles autres que siliconées dans ces compositions tout en conservant une qualité de 'sans transfert' correcte. En effet, les huiles hydrocarbonées, qui sont connues pour apporter notamment du confort à une composition cosmétique, ont comme inconvénient d'augmenter le transfert d'une telle composition.
Il subsiste donc le besoin d'une composition cosmétique qui transfère peu ou pas du tout, c'est-à-dire d'une composition 'sans transfert', tout en possédant de bonnes propriétés cosmétiques.
Notamment il subsiste le besoin d'une composition étant susceptible de comprendre des huiles non siliconés en grande quantité, tout en conservant une très bonne tenue c'est-à-dire en ne transférant pas, ne tachant pas, ou peu, un support avec lequel elle serait en contact, et en ne migrant pas au cours du temps.

Or, à la suite d'études approfondies, la demanderesse a mis en évidence que, de manière inattendue et surprenante, la présence d'un polymère particulier, défini ci-après, dans une composition cosmétique comprenant les constituants usuels de l'art antérieur et des composés volatils, et présentant donc l'inconvénient de transférer, permettait de limiter, voire de supprimer totalement, le transfert et/ou la migration, de ladite composition, et donc permettait d'en améliorer la tenue.
La présente invention a donc trait à une composition de soin ou de maquillage permettant de remédier aux inconvénients de l'art antérieur, permettant en particulier l'obtention d'un film ne transférant pas, ne migrant pas et présentant des propriétés cosmétiques améliorées par rapport à celles des produits 'sans transfert' de l'art antérieur notamment des propriétés de glissant, de non tiraillement, de douceur et de confort.

La présente invention a donc pour objet une composition cosmétique, dermatologique, hygiénique et/ou pharmaceutique en stick, sans transfert, comprenant un composé volatil et un polymère de formule (I), telle que définie dans la revendication 1.
L'invention a également pour objet l'utilisation de l'association d'un composé volatil et d'un polymère de formule (I) dans une composition comme agent pour limiter, diminuer et/ou de supprimer le transfert et/ou la migration de ladite composition.

L'invention a encore pour objet l'utilisation dans une composition de l'association d'au moins un polymère de formule (I) et d'un composé volatil, telle que définie dans la revendication 23, pour limiter, diminuer et/ou empêcher le transfert de ladite composition cosmétique, dermatologique, hygiénique et/ou pharmaceutique, notamment une composition de maquillage ou de soin de la peau, des muqueuses, des semi-muqueuses et/ou des phanères.

La composition selon l'invention trouve notamment une application particulièrement intéressante dans le domaine du soin et/ou du maquillage de la peau, des muqueuses, des semi-muqueuses et des phanères.
On entend notamment par muqueuse, la partie interne de la paupière inférieure; parmi les semi-muqueuses, on entend plus particulièrement les lèvres du visage; par phanères, on entend les cils, sourcils, cheveux et ongles.
Ainsi, l'invention trouve une application toute particulière dans le domaine des produits de maquillage des lèvres du visage, mais aussi des produits de soin des lèvres ainsi que dans le domaine des produits de maquillage et de soin de la peau tels que les fonds de teint, les autobronzants ou les produits solaires.

On a constaté que les compositions comprenant ledit polymère permettent l'obtention d'un film ayant une bonne affinité pour la peau et les muqueuses, ce qui peut se traduire par une meilleure tenue du film dans le temps et une bonne persistance de son homogénéité.
De plus, on a remarqué que la présence dudit polymère permet d'obtenir une bonne dispersion des pigments dans la phase grasse de la composition, ce qui se traduit notamment par une bonne homogénéité de la coloration du maquillage obtenu.
La composition selon l'invention est facilement applicable sur la peau et permet entre autre un maquillage de texture peu collante, qui reste confortable à porter tout au long de la journée.

On a également constaté que la présence d'au moins un polymère tel que défini ci-après, permet d'obtenir une bonne qualité de 'sans transfert', et permet également la réalisation de compositions 'sans transfert' comprenant aussi bien des corps gras siliconés que des corps gras hydrocarbonés ou un mélange de corps gras siliconés et hydrocarbonés.
Ainsi, grâce à l'invention, la présence d'huiles hydrocarbonées, et notamment d'huiles végétales, connues pour apporter du confort, de l'onctuosité et/ou de la couvrance, est rendue possible, tout en conservant un 'sans transfert' remarquable.

Le polymère utilisé dans le cadre de la présente invention peut donc être défini comme étant un dérivé de vinylpyrrolidone, plus précisément soit un copolymère de polyvinylpyrrolidone et d'a-oléfines, soit un dérivé alkylé de polyvinylpyrrolidone. Ces polymères sont lipophiles.
Ils peuvent notamment être représentés par la formule (I) suivante : 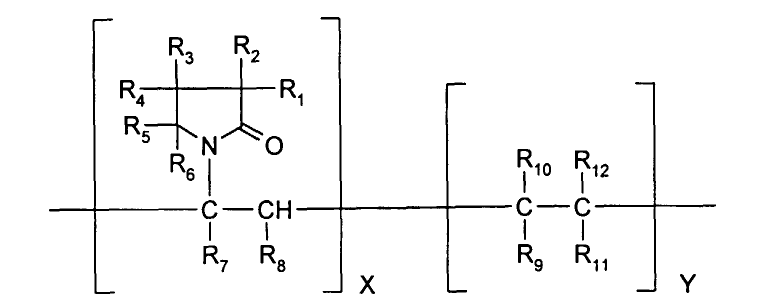 dans laquelle les radicaux R1 à R12 représentent, indépendamment l'un de l'autre, un radical alkyle saturé à chaîne droite ou ramifiée en C10 à C40 ou l'atome d'hydrogène, l'un au moins desdits radicaux R1 à R12 étant différent de l'atome d'hydrogène.
La valeur y peut être égale à 0; x est obligatoirement non nul.

De préférence, le polymère utilisé dans le cadre de l'invention présente au moins un radical R comprenant 14 à 32 atomes de carbone, préférentiellement 28 à 32 atomes de carbone.
Parmi les radicaux alkyle comprenant 10 à 40 atomes de carbone, on peut citer les radicaux pentadécyle, hexadécyle, heptadécyle, octadécyle, nonadécyle, eicosyle, docosyle et triacontyle.
De préférence, la masse moléculaire en poids des polymères selon l'invention est comprise entre 5000 et 30 000, en particulier entre 6000 et 20 000.

Dans un mode de réalisation particulier de l'invention, y est égal à 0 et les radicaux R2 à R5 représentent l'hydrogène. De préférence, au moins un des radicaux différents de l'hydrogène comprend de 14 à 32 atomes de carbone
Parmi les polymères répondant à ce mode de réalisation, on peut citer le triacontanyl-PVP (nom CTFA : tricontanyl PVP). Parmi les produits commerciaux, on peut citer les produits commercialisés par la société GAF sous la dénomination ANTARON WP660.

Dans un autre mode de réalisation de l'invention, y peut être non nul. Les radicaux R1 à R9 et R11 et R12 représentent de préférence l'hydrogène. De préférence R10 comprend de 14 à 32 atomes de carbone, et le rapport x/y est de préférence compris entre 1/5 et 5/1.

Parmi les polymères répondant à ce mode de réalisation, on peut citer le copolymère PVP/hexadécène (nom CTFA) ou le copolymère PVP/eicosène (nom CTFA).
Parmi les produits commerciaux, on peut citer les produits commercialisés par la société GAF sous la dénomination ANTARON V-216 qui est un copolymère PVP/hexadécène comprenant environ 15-23% de motif pyrrolidone, de poids moléculaire 7300; ou sous la dénomination ANTARON V-220 qui est un copolymère PVP/eicosène qui comprend environ 20-28% en poids de motif pyrrolidone et qui a un poids moléculaire en masse de 8600.

Le polymère selon l'invention possède une consistance à température ambiante pouvant être plus ou moins visqueuse, selon la longueur de la chaîne alkyle. Il peut ainsi se présenter sous forme liquide ayant une viscosité de l'ordre de 40 à 55 poises (4 à 5,5 Pa.s), sous forme plus pâteuse ou encore sous forme solide proche de la consistance d'une cire.

Le polymère peut être présent dans la composition à raison de 0,5 à 40% en poids, de préférence 1 à 20% en poids.
On peut utiliser, seul ou en mélange, des polymères se présentant sous forme liquide, pâteuse et/ou solide. Ces polymères peuvent notamment remplacer une partie de la phase grasse de la composition.
Par exemple, il est possible de remplacer tout ou partie des cires usuellement utilisées par un polymère de consistance semblable; il est également possible de remplacer une partie des huiles siliconées et/ou hydrocarbonées par un polymère se présentant sous forme liquide.

La composition selon l'invention comprenant au moins un polymère tel que défini ci-dessus peut comprendre par ailleurs tout composé connu de l'homme du métier pour le type d'application envisagé.

En particulier, la composition comprend des composés volatils à température ambiante (20-25°C). Par composé volatil, on entend dans la présente description, tout composé susceptible de s'évaporer au contact de la peau. De préférence, on utilise des huiles volatiles dont le point éclair est suffisamment élevé pour permettre l'utilisation de ces huiles en formulation, et suffisamment bas pour obtenir l'effet évanescent souhaité. On emploie de préférence des huiles volatiles dont le point éclair est de l'ordre de 40-100°C.
Ces composés volatils peuvent être choisis en particulier parmi les huiles hydrocarbonées et/ou les huiles siliconées, cycliques ou linéaires, seules ou en mélange.
Parmi les huiles siliconées volatiles, on peut citer, seules ou en mélange,
- les silicones volatiles cycliques ayant de 3 à 8 atomes de silicium et de préférence de 4 à 6. Il s'agit par exemple de la cyclotétradiméthylsiloxane, de la cyclopentadiméthylsiloxane ou de la cyclohexadiméthylsiloxane,
- les cyclocopolymères du type diméthylsiloxane/méthylalkylsiloxane, tels que la SILICONE FZ 3109 vendue par la société UNION CARBIDE , qui est un cyclocopolymère diméthylsiloxane/méthyloctylsiloxane,
- les silicones volatiles linéaires ayant de 2 à 9 atomes de silicium. Il s'agit par exemple de l'hexaméthyldisiloxane ou un PDMS de faible viscosité (1 cSt). On peut encore citer les alkyltrisiloxanes tels que l'hexylheptaméthyltrisiloxane ou l'octylheptaméthyltrisiloxane.
Parmi les huiles hydrocarbonées volatiles, on peut citer les isoparaffines. et notamment l'isododécane
La composition selon l'invention peut comprendre 8 à 70 % en poids, de préférence 30 à 60 % en poids, de composés volatils, par rapport au poids total de la composition.

La composition peut également comprendre des corps gras non volatils usuellement utilisés dans le domaine d'application envisagé, tels que des huiles, des gommes, des corps gras pâteux et/ou des cires d'origine végétale, minérale, animale, synthétique, siliconée et/ou fluorée. Ces corps gras peuvent en particulier être choisis de manière variée par l'homme du métier afin de préparer une composition ayant les propriétés souhaitées, par exemple en consistance ou en texture.

L'utilisation d'un polymère selon l'invention en association avec un composé volatil permet d'obtenir une propriété de 'sans transfert' remarquable, quelle que soit la nature des corps gras optionnels. Ainsi, ces corps gras peuvent être aussi bien hydrocarbonés que siliconés, ce qui permet d'adapter les propriétés du film notamment en ce qui concerne le confort sur les lèvres ou la peau des êtres humains. Les adjuvants doivent bien entendu ne pas nuire à l'homogénéité et à la stabilité de la composition.

Ainsi, la composition selon l'invention comprend au moins une cire, qui peut assurer notamment la résistance mécanique de la composition, lorsqu'elle se présente sous la forme d'un stick.

On peut employer toute cire connue dans l'art antérieur parmi lesquelles, seules ou en mélange, on peut citer les cires animales, végétales, minérales et synthétiques telles que les cires microcristallines, la paraffine, le pétrolatum, la vaseline, l'ozokérite, la cire de montan; la cire d'abeilles, la lanoline et ses dérivés; les cires de Candellila, d'Ouricury, de Carnauba, du Japon, le beurre de cacao, les cires de fibres de lièges ou de canne à sucre; les huiles hydrogénées concrètes à 25°C, les ozokérites, les esters gras et les glycérides concrets à 25°C; les cires de polyéthylène et les cires obtenues par synthèse de Fischer-Tropsch; les cires de silicone; les cires fluorées.
De préférence, les cires entrant dans la composition peuvent présenter un point de fusion supérieur à 45°C environ, et en particulier supérieur à 55°C, et/ou un indice de pénétration de l'aiguille à 25°C de préférence compris entre 3 et 40.
De préférence, la composition comprend 0,5 à 30% en poids de cire, notamment 5 à 20% en poids.

La composition selon l'invention peut également comprendre au moins une huile siliconée, fluorée et/ou hydrocarbonée, ou un mélange de ces différentes huiles.
Parmi les huiles siliconées, on peut citer les huiles de silicone phénylées, notamment de type polyphénylméthylsiloxane ou phényltriméthicone, et en particulier les huiles répondant à la formule suivante : 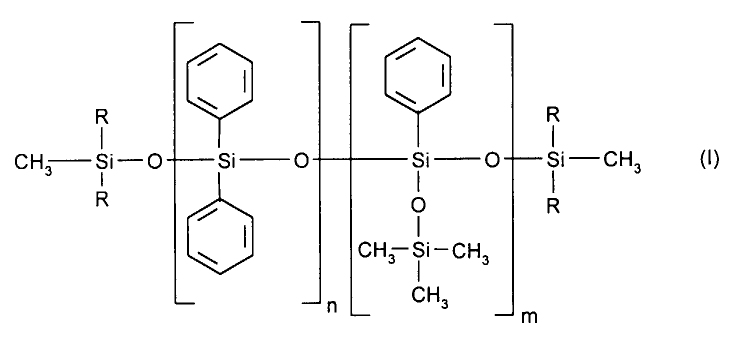 dans laquelle
. R est un radical alkyle en C1-C30, un radical aryle ou un radical aralkyle,
. n est un nombre entier compris entre 0 et 100,
. m est un nombre entier compris entre 0 et 100, sous réserve que la somme m+n est comprise entre 1 et 100.
On peut encore citer les polyalkyl(C₁-C₂₀) siloxanes et notamment ceux à groupements terminaux triméthylsilyle, parmi lesquels on peut citer les polydiméthylsiloxanes linéaires et les alkylméthylpolysiloxanes; les alkyldiméthicones; les silicones modifiées par des groupements aliphatiques et/ou aromatiques, éventuellement fluorés, ou par des groupements fonctionnels tels que des groupements hydroxyles, thiols et/ou amines.

Parmi les huiles hydrocarbonées d'origine animale, végétale ou minérale, on peut citer l'huile de paraffine, de vaseline, le perhydrosqualène, l'huile d'arachide, d'amande douce, de macadamia, de pépins de raisin, de colza, de coprah, d'arachide, de calophyllum, de palme, de ricin, d'avocat, d'abricot, de sésame, de jojoba, d'olive ou de germes de céréales; des esters d'acides gras; des alcools; des acétylglycérides; des octanoates, décanoates ou ricinoléates d'alcools ou de polyalcools; des triglycérides d'acides gras; des glycérides, leurs mélanges.
On peut encore citer les huiles fluorées ou perfluorées, telles que les perfluoropolyéthers ou les perfluoroalcanes.

De préférence, la composition selon l'invention peut comprendre 0 à 45% en poids d'huiles, notamment 20 à 40% en poids. Dans un mode de réalisation préféré, toutes les huiles présentes dans la composition sont hydrocarbonées.

Parmi les corps gras susceptibles d'être présents dans la composition, on peut encore citer les gommes de silicone ainsi que les corps gras pâteux.

Dans un mode préféré de réalisation, la composition peut comprendre uniquement des corps gras hydrocarbonés, et notamment des cires de polyéthylène en association avec des huiles végétales. Le polymère présent dans cette composition peut être liquide, pâteux et/ou solide, de manière à obtenir la consistance finale souhaitée. De préférence, le polymère choisi présente, de manière prédominante, un radical R comprenant 28 à 32 atomes de carbone.

La composition selon l'invention peut comprendre en outre une partie hydrophile, qui peut se présenter, par exemple, sous forme d'un alcool polyhydrique et/ou sous forme une phase aqueuse épaissie, voire gélifiée.
L'alcool polyhydrique peut être un composé ayant 2-8 atomes de carbone et 2-6 fonctions hydroxyles, tel que l'éthylène glycol, le glycérol, le propane-1,2 diol, la diglycérine, l'érythritol, l'arabitol, l'adonitol, le sorbitol et le dulcitol. L'alcool polyhydrique peut également être un polyéther alcool de poids moléculaire moyen de 150-600, tel que le polyéthylène glycol 300 et la polyglycérine 500. On peut également employer un mélange d'alcools polyhydriques.
La phase aqueuse épaissie ou gélifiée, peut comprendre de l'eau et/ou une eau florale telle que l'eau de bleuet, et/ou une eau minérale telle que l'eau de VITTEL, l'eau de LUCAS ou l'eau de LA ROCHE POSAY.
Elle peut être épaissie par la présence de tout agent épaississant connu de l'homme du métier. On peut notamment citer :
. les extraits d'algues tels que l'agar-agar, les carraghénanes et les alginates,
. les extraits de graines tels que les extraits de caroube ou de guar,
. les extraits de fruits, notamment la pectine,
. les exsudats de plantes, tels que la gomme arabique, l'adragante, la gomme de karaya et la gomme de ghatty,
. les dérivés cellulosiques, tels que la carboxyméthylcellulose,
. les gélifiant d'origine animale tels que la gélatine ou les caseinates,
. les exsudats de micro-organismes tels que la gomme xanthane,
. les gélifiants synthétiques tels que les dérivés de polymère acrylique (Carbomer, Carbopol, Pemulen) ou les dérivés du silicium (Laponite, Lapomer ou Veegum).
L'agent épaississant peut être présent dans la phase aqueuse à raison de 0,2-10% en poids par rapport à la phase aqueuse.
En outre, ladite partie hydrophile peut comprendre des additifs hydrosolubles, notamment des vitamines, des actifs cosmétiques et/ou pharmaceutiques, des colorants.
La partie hydrophile peut représenter 0 à 40% en poids de la composition, de préférence 8 à 20% en poids.

La composition peut comprendre également une phase particulaire, qui peut comprendre des pigments et/ou des nacres et/ou des charges habituellement utilisés dans les compositions cosmétiques.
Par pigments, il faut comprendre des particules blanches ou colorées, minérales ou organiques, destinées à colorer et/ou opacifier la composition. Par charges, il faut comprendre des particules incolores ou blanches, minérales ou de synthèse, lamellaires ou non lamellaires, destinées à donner du corps ou de la rigidité à la composition, et/ou de la douceur, de la matité et de l'uniformité au maquillage.
Par nacres, il faut comprendre des particules irisées qui réfléchissent la lumière.
Les pigments peuvent être présents dans la composition à raison de 0 à 15% en poids de la composition finale, et de préférence à raison de 8 à 10% en poids. Ils peuvent être blancs ou colorés, minéraux et/ou organiques, de taille usuelle ou nanométrique.
On peut citer les dioxydes de titane, de zirconium ou de cérium, ainsi que les oxydes de zinc, de fer ou de chrome, le bleu ferrique, le noir de carbone, les outremers (polysulfures d'aluminosilicates), le pyrophosphate de manganèse et certaines poudres métalliques telles que celles d'argent ou d'aluminium. On peut encore citer les laques couramment employées pour conférer aux lèvres et à la peau un effet de maquillage, qui sont des sels de calcium, de baryum, d'aluminium ou de zirconium, de colorants acides tels que les colorants halogéno-acides, azoïques, anthraquinoniques, etc.
Les nacres peuvent être présentes dans la composition à raison de 0 à 20% en poids, de préférence à un taux de l'ordre de 8 à 15% en poids. Parmi les nacres envisageables, on peut citer la nacre naturelle, le mica recouvert d'oxyde de titane, d'oxyde de fer, de pigment naturel ou d'oxychlorure de bismuth ainsi que le mica titane coloré.
Les charges, qui peuvent être présentes à raison de 0 à 30% en poids, de préférence 5 à 15%, dans la composition, peuvent être minérales ou de synthèse, lamellaires ou non lamellaires. On peut citer le talc, le mica, la silice, le kaolin, les poudres de Nylon et de polyéthylène, le Téflon, l'amidon, le nitrure de bore, les microsphères telles que l'Expancel (Nobel Industrie), le polytrap (Dow Corning) et les microbilles de résine de silicone (Tospearls de Toshiba, par exemple).

La composition peut comprendre en outre tout additif usuellement utilisé dans le domaine cosmétique, tel que des antioxydants, des parfums, des huiles essentielles, des conservateurs, des actifs cosmétiques lipophiles ou hydrophiles, des hydratants, des vitamines, des colorants, des acides gras essentiels, des sphingolipides, des agents autobronzants tels que la DHA, des filtres solaires.
Bien entendu l'homme du métier veillera à choisir les éventuels composés complémentaires, et/ou leur quantité, de manière telle que les propriétés avantageuses de la composition selon l'invention ne soient pas, ou substantiellement pas, altérées par l'adjonction envisagée. Ces additifs peuvent être présents dans la composition à raison de 0-10% en poids.

La composition selon l'invention peut également comprendre au moins un agent actif, parmi lesquels on peut citer les agents actifs contre les micro-organismes, notamment à activité antivirale, antibactérienne ou antifongique; les agents à activité anti-inflammatoire ou immunomodulatrice; les agents antagonistes des neuromédiateurs ou modulant le relarguage des neuromédiateurs; les agents modulant la différenciation et/ou la prolifération cellulaire et/ou la pigmentation et/ou régulant la kératinisation; les agents actifs dans le traitement et/ou la prévention des cheilites; les antihistaminiques; les agents cicatrisants.
Selon les pathologies à traiter, il peut être judicieux d'associer plusieurs agents actifs, ou encore de leur adjoindre des actifs secondaires qui vont favoriser la prévention ou le traitement de l'affection, et/ou traiter des symptômes associés à cette affection. Parmi les agents actifs secondaires, on peut citer les anesthésiques locaux, les antiseptiques, les hydratants et/ou émollients, et les filtres solaires notamment chimiques ou minéraux.
La composition peut en outre contenir un agent favorisant la solubilisation ou la compatibilité des agents actifs avec l'excipient de la composition, et/ou favorisant la pénétration desdits agents actifs dans la peau ou les muqueuses. Il peut s'agir par exemple du myristate d'isopropyle, de l'acide oléique, de la lécithine ou de certains alcools.

La composition selon l'invention se présente sous la forme d'un stick ou bâton.

Les compositions selon l'invention trouvent une application notamment dans le domaine du maquillage de la peau, des semi-muqueuses, des muqueuses et/ou des phanères, et se présentent alors par exemple sous la forme d'un fond de teint, d'un fard à joues ou à paupières, d'un rouge à lèvres, d'un mascara ou d'un eye-liner.

Elles peuvent également se présenter sous forme non colorée, contenant éventuellement des actifs cosmétiques. Elles peuvent alors notamment être utilisées comme base de soin pour les lèvres ou comme base fixante à appliquer sur un rouge à lèvres classique. La base fixante forme alors un film protecteur sur le film de rouge, en limite le transfert et la migration, et permet d'augmenter ainsi sa tenue. Les compositions selon l'invention peuvent également se présenter sous la forme d'un produit de soin de la peau, des muqueuses, des semi-muqueuses et/ou des phanères, d'un produit hygiénique ou pharmaceutique ou encore d'un produit solaire ou autobronzant.

L'invention est illustrée plus en détail dans les exemples suivants.

### Exemple 1

On prépare un bâton de rouge à lèvres ayant la composition suivante
. PVP triacontène (ANTARON WP660 de GAF) 2 g
. cire de polyéthylène 25 g
. huile végétale 23,5 g
. pigments 9,5 g
. cyclotétrapolysiloxane qsp 100 g
On prépare la composition de manière usuelle, en chauffant la cire, l'huile végétale et le polymère à 95°C et en les mélangeant. On ajoute les pigments et, à 60°C, l'huile volatile siliconée. On mélange le tout à l'aide d'une turbine Moritz à la vitesse de 3000 tr/min. On peut alors couler le mélange homogène obtenu dans des moules adéquats.
On obtient après refroidissement, un bâton de rouge à lèvres de texture agréable, qui s 'applique uniformément sur les lèvres et dont le film est très confortable, tout en ne laissant pas de traces sur des supports extérieurs après évaporation de l'huile volatile (quelques minutes). Ceci est d'autant plus remarquable que la composition comprend une quantité importante d'huile hydrocarbonée, peu favorable au 'sans transfert'.

On applique cette composition sur la partie gauche des lèvres de plusieurs personnes.
Pour comparaison, on applique sur la partie droite desdites lèvres, un rouge à lèvres 'sans transfert' de l'art antérieur, ne comprenant pas de polymère.
On laisse sécher les rouges à lèvres à température ambiante pendant 30 minutes, puis on applique la totalité des lèvres sur une feuille de papier.
On constate sur la totalité des feuilles de papier une trace de rouge à lèvres très faible, à peine perceptible, aussi bien pour la composition de l'invention que pour la composition de l'art antérieur.

### Exemple 2

On prépare un bâton de rouge à lèvres ayant la composition suivante
. PVP/eicosène (ANTARON V220 de GAF) 2 g
. cire de polyéthylène 20 g
. huile végétale 28,5 g
. pigments 9,5 g
. cyclotétrapolysiloxane qsp 100 g
On prépare la composition selon l'exemple 1.
On obtient un bâton de rouge à lèvres présentant à la fois des propriétés de 'sans transfert' remarquables et permettant l'obtention d'un film confortable.

### Exemple 3

On prépare un bâton de rouge à lèvres ayant la composition suivante
. PVP/triacontène (ANTARON WP660 de GAF) 2 g
. cire de polyéthylène 20 g
. malate de diisostéaryle 28,5 g
. pigments 9,5 g
. cyclotétrapolysiloxane qsp 100 g
On prépare la composition selon l'exemple 1.
On obtient un bâton de rouge à lèvres présentant à la fois des propriétés de 'sans transfert' remarquables et permettant l'obtention d'un film confortable.

## Revendications

1. Stick cosmétique, dermatologique, hygiénique et/ou pharmaceutique, sans transfert, comprenant de 30 à 60 % en poids d'un composé volatil, par rapport au poids total de la composition, au moins une cire et et un polymère de formule (I): 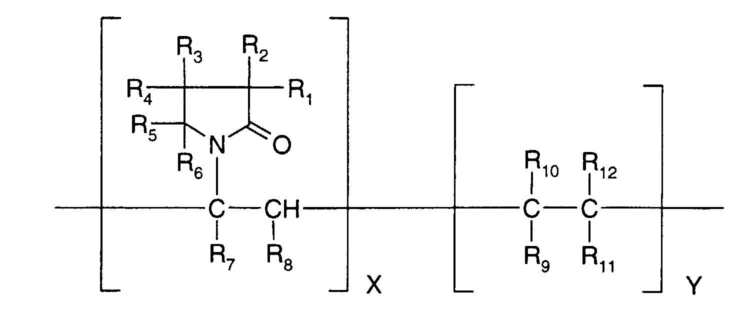 dans laquelle les radicaux R1 à R12 représentent, indépendamment l'un de l'autre, un radical alkyle saturé à chaîne droite ou ramifiée en C10 à C40 ou l'atome d'hydrogène, l'un au moins desdits radicaux R1 à R12 étant différent de l'atome d'hydrogène; x étant non nul.

2. Composition selon la revendication 1, dans laquelle l'un au moins des radicaux R1 à R12 représente un radical alkyle comprenant 14 à 32 atomes de carbone, de préférence 28 à 32 atomes de carbone.

3. Composition selon l'une des revendications précédentes, dans laquelle le polymère possède une masse moléculaire en poids comprise entre 5000 et 30 000, en particulier entre 6000 et 20 000.

4. Composition selon l'une des revendications précédentes, dans laquelle y est égal à 0 et les radicaux R2 à R5 représentent l'hydrogène.

5. Composition selon la revendication 4, dans laquelle au moins un des radicaux différents de l'hydrogène comprend de 14 à 32 atomes de carbone.

6. Composition selon l'une des revendications 1-3, dans laquelle y est non nul et les radicaux R1 à R9 et R11 et R12 représentent l'hydrogène.

7. Composition selon la revendication 6, dans laquelle R10 comprend de 14 à 32 atomes de carbone, et le rapport x/y est de préférence compris entre 1/5 et 5/1.

8. Composition selon l'une des revendications précédentes, dans laquelle le polymère est choisi parmi le copolymère PVP/hexadécène, le copolymère PVP/eicosène, le polymère triacontanyl-PVP et leurs mélanges.

9. Composition selon l'une des revendications précédentes, dans laquelle le polymère est présent dans la composition à raison de 0,5 à 40% en poids, de préférence 1 à 20% en poids.

10. Composition selon l'une des revendications précédentes, dans laquelle le composé volatil est choisi parmi les huiles hydrocarbonées et/ou les huiles siliconées, cycliques ou linéaires, seules ou en mélange.

11. Composition selon l'une des revendications précédentes, dans laquelle le composé volatil est choisi parmi les silicones volatiles cycliques ayant de 3 à 8 atomes de silicium, de préférence de 4 à 6; les cyclocopolymères du type diméthylsiloxane/méthylalkylsiloxane; les silicones volatiles linéaires ayant de 2 à 9 atomes de silicium; les alkyltrisiloxanes; les isoparaffines; leurs mélanges.

12. Composition selon l'une des revendications précédentes, comprenant en outre au moins un corps gras non volatil choisi parmi les huiles, les gommes, les corps gras pâteux et/ou les cires d'origine végétale, minérale, animale, synthétique, siliconée et/ou fluorée.

13. Composition selon l'une des revendications précédentes, dans laquelle la cire est choisie parmi les cires microcristallines, la paraffine, le pétrolatum, la vaseline, l'ozokérite, la cire de montan; la cire d'abeilles, la lanoline et ses dérivés; les cires de Candellila, d'Ouricury, de Carnauba, du Japon, le beurre de cacao, les cires de fibres de lièges ou de canne à sucre; les huiles hydrogénées concrètes à 25°C, les ozokérites, les esters gras et les glycérides concrets à 25°C; les cires de polyéthylène et les cires obtenues par synthèse de Fischer-Tropsch; les cires de silicone; les cires fluorées, leurs mélanges.

14. Composition selon l'une des revendications précédentes, comprenant en outre au moins une huile de silicone phénylée répondant à la formule suivante : 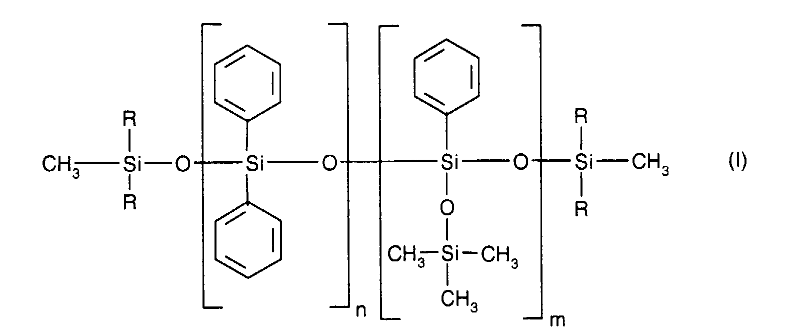 dans laquelle
. R est un radical alkyle en C1-C30, un radical aryle ou un radical aralkyle,
. n est un nombre entier compris entre 0 et 100,
. m est un nombre entier compris entre 0 et 100, sous réserve que la somme m+n est comprise entre 1 et 100.

15. Composition selon l'une des revendications précédentes, comprenant en outre au moins une huile hydrocarbonée choisie parmi l'huile de paraffine, de vaseline, le perhydrosqualène, l'huile d'arachide, d'amande douce, de macadamia, de pépins de raisin, de colza, de coprah, d'arachide, de calophyllum, de palme, de ricin, d'avocat, d'abricot, de sésame, de jojoba, d'olive ou de germes de céréales; des esters d'acides gras; des alcools; des acétylglycérides; des octanoates, décanoates ou ricinoléates d'alcools ou de polyalcools; des triglycérides d'acides gras; des glycérides, leurs mélanges.

16. Composition selon la revendication 15, dans laquelle la teneur en huile hydrocarbonée est comprise entre 0 et 45% en poids, de préférence 20 à 40% en poids.

17. Composition selon l'une des revendications précédentes, comprenant en outre une partie hydrophile choisie parmi au moins un alcool polyhydrique et/ou une phase aqueuse épaissie, voire gélifiée.

18. Composition selon la revendication 17, dans laquelle la partie hydrophile représente 0 à 40% en poids de la composition, de préférence 8 à 20% en poids.

19. Composition selon l'une des revendications précédentes, comprenant en outre une phase particulaire comprenant des pigments et/ou des nacres et/ou des charges.

20. Composition selon l'une des revendications précédentes, comprenant en outre au moins un agent actif choisi parmi les agents actifs contre les micro-organismes; les agents à activité anti-inflammatoire ou immunomodulatrice; les agents antagonistes des neuromédiateurs ou modulant le relarguage des neuromédiateurs; les agents modulant la différenciation et/ou la prolifération cellulaire et/ou la pigmentation et/ou régulant la kératinisation; les agents actifs dans le traitement et/ou la prévention des cheilites; les antihistaminiques; les agents cicatrisants.

21. Composition selon l'une des revendications précédentes, se présentant sous la forme d'un rouge à lèvres, un fond de teint, un fard à joues ou à paupières, un mascara, un soin des lèvres, un autobronzant et/ou un produit solaire.

22. Utilisation dans une composition cosmétique ou hygiénique ou pour la fabrication d'une composition dermatologique ou pharmaceutique de l'association d'un composé volatil et d'un polymère de formule I : 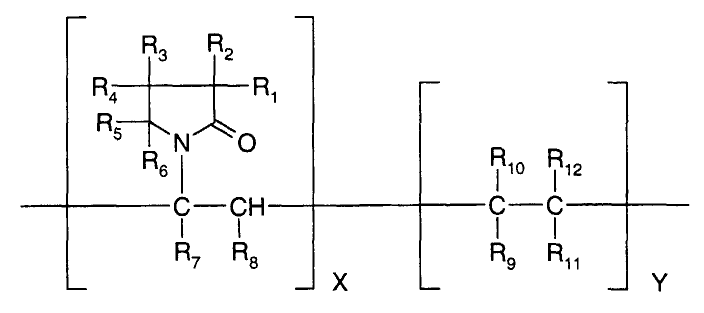 dans laquelle les radicaux R1 à R12 représentent, indépendamment l'un de l'autre, un radical alkyle saturé à chaîne droite ou ramifiée en C10 à C40 ou l'atome d'hydrogène, l'un au moins desdits radicaux R1 à R12 étant différent de l'atome d'hydrogène; x étant non nul, comme agent pour limiter, diminuer et/ou supprimer le transfert et/ou la migration de ladite composition.

23. Utilisation dans une composition cosmétique ou hygiénique ou pour la fabrication d'une composition dermatologique ou pharmaceutique de 30 à 60 % en poids, par rapport au poids total de la composition, d'un composé volatil et d'un polymère de formule (I) 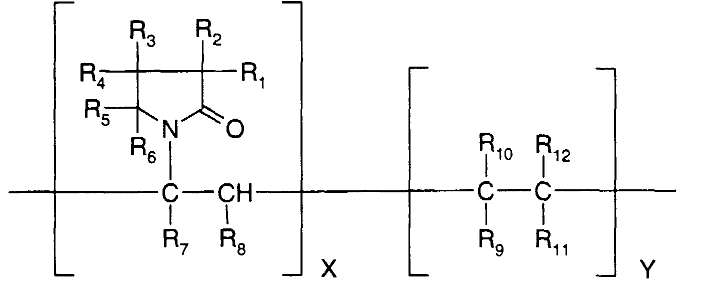 dans laquelle les radicaux R1 à R12 représentent, indépendamment l'un de l'autre, un radical alkyle saturé à chaîne droite ou ramifiée en C10 à C40 ou l'atome d'hydrogène, l'un au moins desdits radicaux R1 à R12 étant différent de l'atome d'hydrogène; x étant non nul, pour limiter, diminuer et/ou supprimer le transfert et/ou la migration de ladite composition.

24. Utilisation selon la revendication 22, dans laquelle la composition comprend 8 à 70 % en poids, de préférence 30 à 60 % en poids, de composé volatil, par rapport au poids total de la composition.

25. Utilisation selon l'une des revendications 22 à 24, dans laquelle au moins un des radicaux différents de l'hydrogène comprend de 14 à 32 atomes de carbone, de préférence 28 à 32 atomes de carbone.

26. Utilisation selon l'une des revendications 22 à 25, dans laquelle y est égal à 0, les radicaux R2 à R5 représentent l'hydrogène et au moins un des radicaux différents de l'hydrogène comprend de 14 à 32 atomes de carbone.

27. Utilisation selon l'une des revendications 22 à 26, dans laquelle y est non nul, les radicaux R1 à R9 et R11 et R12 représentent l'hydrogène, le radical R10 comprend de 14 à 32 atomes de carbone, et le rapport x/y est de préférence compris entre 1/5 et 5/1.

28. Utilisation selon l'une des revendications 22 à 27, dans laquelle le polymère est choisi parmi le copolymère PVP/hexadécène, le copolymère PVP/eicosène, le polymère triacontanyl-PVP et leurs mélanges.

29. Utilisation selon l'une des revendications 22 à 28, dans laquelle le polymère est présent dans la composition à raison de 0,5 à 40% en poids, de préférence 1 à 20% en poids.

30. Utilisation selon l'une des revendications 22 à 29, dans laquelle le composé volatil est choisi parmi les huiles hydrocarbonées et/ou les huiles siliconées, cycliques ou linéaires, seules ou en mélange.

31. Utilisation selon l'une des revendications 22 à 30, dans laquelle la composition comprend en outre au moins un corps gras non volatil choisi parmi les huiles, les gommes, les corps gras pâteux et/ou les cires d'origine végétale, minérale, animale, synthétique, siliconée et/ou fluorée.

32. Utilisation selon l'une des revendications 22 à 31, dans laquelle la composition comprend 0 à 45% en poids d'huile hydrocarbonée, de préférence 20 à 40% en poids.

33. Utilisation selon l'une des revendications 22 à 32, dans laquelle la composition comprend en outre une partie hydrophile choisie parmi au moins un alcool polyhydrique et/ou une phase aqueuse épaissie, voire gélifiée.

34. Utilisation selon l'une des revendications 22 à 33, dans un rouge à lèvres, un fond de teint, un fard à joues ou à paupières, un mascara, un eye-liner, un soin des lèvres, un autobronzant et/ou un produit solaire.

## Patentansprüche

1. Kosmetischer, dermatologischer, hygienischer und/oder pharmazeutischer Stift 'ohne Transfer', der 30 bis 60 Gew.-% einer flüchtigen Verbindung, bezogen auf das Gesamtgewicht der Zusammensetzung, mindestens ein Wachs und ein Polymer der Formel (I) enthält: 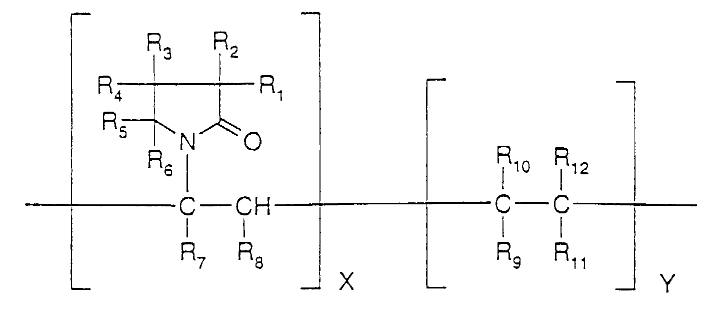 worin die Gruppen R₁ bis R₁₂ unabhängig voneinander eine gesättigte, geradkettige oder verzweigte C₁₀₋₄₀-Alkylgruppe oder ein Wasserstoffatom bedeuten, wobei mindestens eine der Gruppen R₁ bis R₁₂ von Wasserstoff verschieden ist und x nicht 0 ist.

2. Zusammensetzung nach Anspruch 1, wobei eine der Gruppen R₁ bis R₁₂ eine Alkylgruppe mit 14 bis 32 Kohlenstoffatomen und vorzugsweise 28 bis 32 Kohlenstoffatomen bedeutet.

3. Zusammensetzung nach einem der vorhergehenden Ansprüche, wobei das Polymer ein Gewichtsmittel des Molekulargewichts im Bereich von 5 000 bis 30 000 und insbesondere 6 000 bis 20 000 aufweist.

4. Zusammensetzung nach einem der vorhergehenden Ansprüche, wobei y 0 ist und die Gruppen R₂ bis R₅ Wasserstoff bedeuten.

5. Zusammensetzung nach Anspruch 4, wobei mindestens eine der Gruppen, die von Wasserstoff verschieden sind, 14 bis 32 Kohlenstoffatome aufweist.

6. Zusammensetzung nach einem der Ansprüche 1 bis 3, wobei y nicht 0 ist und die Gruppen R₁ bis R₉ und R₁₁ und R₁₂ Wasserstoffbedeuten.

7. Zusammensetzung nach Anspruch 6, wobei R₁₀ 14 bis 32 Kohlenstoffatome aufweist und das Verhältnis x/y vorzugsweise im Bereich von 1/5 bis 5/1 liegt.

8. Zusammensetzung nach einem der vorhergehenden Ansprüche, wobei das Polymer unter dem Copolymer PVP/Hexadecen, dem Copolymer PVP/Eicosen, dem Polymer Triacontanyl-PVP und deren Gemischen ausgewählt ist.

9. Zusammensetzung nach einem der vorhergehenden Ansprüche, wobei das Polymer in der Zusammensetzung in Mengenanteilen von 0,5 bis 40 Gew.-% und vorzugsweise 1 bis 20 Gew.-% vorliegt.

10. Zusammensetzung nach einem der vorhergehenden Ansprüche, wobei die flüchtige Verbindung unter den Kohlenwasserstoffölen und/oder den cyclischen oder linearen Siliconölen oder deren Gemischen ausgewählt ist.

11. Zusammensetzung nach einem der vorhergehenden Ansprüche, wobei die flüchtige Verbindung unter den flüchtigen cyclischen Siliconen mit 3 bis 8 und vorzugsweise 4 bis 6 Siliciumatomen; den Cyclocopolymeren vom Typ Dimethylsiloxan/ Methylalkylsiloxan; den flüchtigen linearen Siliconen mit 2 bis 9 Siliciumatomen; den Alkyltrisiloxanen; den Isoparaffinen und deren Gemischen ausgewählt ist.

12. Zusammensetzung nach einem der vorhergehenden Ansprüche, wobei sie femer mindestens eine nichtflüchtige Fettsubstanz enthält, die unter den Ölen, Gummis, pastösen Fettsubstanzen und/oder Wachsen, die pflanzlich, mineralisch, tierisch, synthetisch, siliconhaltig und/oder fluoriert sein können, ausgewählt ist.

13. Zusammensetzung nach einem der vorhergehenden Ansprüche, wobei das Wachs unter den mikrokristallinen Wachsen, Paraffin, Petrolatum, Vaseline, Ozokerit, Montanwachs; Bienenwachs, Lanolin und sein Derivaten; Candelillawachs, Ouricurywachs, Carnaubawachs, Japanwachs, Cacaobutter, Wachsen aus Korkfasern oder Zuckerrohr; bei 25 °C festen, hydrierten Ölen; Ozokeriten, Fettsäureestern und Glyceriden, die bei 25 °C fest sind, Polyethylenwachsen und durch Fischer-Tropsch-Synthese hergestellten Wachsen; Siliconwachsen; fluorierten Wachsen und deren Gemischen ausgewählt ist.

14. Zusammensetzung nach einem der vorhergehenden Ansprüche, die ferner mindestens ein phenylgruppen-haltiges Siliconöl enthält, das der folgenden Formel entspricht: 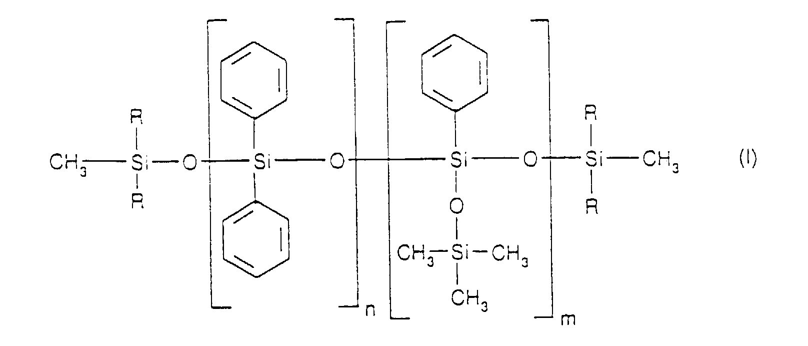 worin bedeuten:
- R eine C₁₋₃₀-Alkylgruppe, eine Arylgruppe oder eine Aralkylgruppe,
- n Null oder eine ganze Zahl im Bereich von 1 bis 100, und
- m Null oder eine ganze Zahl im Bereich von 1 bis 100, mit der Maßgabe, daß n + m im Bereich von 1 bis 100 liegt.

15. Zusammensetzung nach einem der vorhergehenden Ansprüche, die ferner mindestens ein Kohlenwasserstofföl enthält, das unter Paraffinöl, Vaselineöl, Perhydrosqualen, Erdnußöl, Süßmandelöl, Macadamiaöl, Traubenkernöl, Rapsöl, Koprahöl, Calophyllumöl, Palmöl, Ricinusöl, Avocadoöl, Aprikosenöl, Sesamöl, Jojobaöl, Olivenöl und Getreidekeimöl; Fettsäureestem; Alkoholen; Acetylglyceriden; Octanoaten, Decanoaten oder Ricinoleaten von Alkoholen oder Polyalkoholen; Triglyceriden von Fettsäuren; Glyceriden und deren Gemischen ausgewählt ist.

16. Zusammensetzung nach Anspruch 15, wobei der Gehalt an Kohlenwasserstofföl im Bereich von 0 bis 45 Gew.-% und vorzugsweise 20 bis 40 Gew.-% liegt.

17. Zusammensetzung nach einem der vorhergehenden Ansprüche, die ferner mindestens eine hydrophile Komponente enthält, die unter mindestens einem mehrwertigen Alkohol und/oder einer dickflüssigen oder sogar gelierten wäßrigen Phase ausgewählt ist.

18. Zusammensetzung nach Anspruch 17, wobei die hydrophile Komponente 0 bis 40 Gew.-% der Zusammensetzung und vorzugsweise 8 bis 20 Gew.-% ausmacht.

19. Zusammensetzung nach einem der vorhergehenden Ansprüche, die ferner eine Partikelphase aufweist, die Pigmente und/oder Perlglanzpigmente und/oder Füllstoffe enthält.

20. Zusammensetzung nach einem der vorhergehenden Ansprüche, die ferner mindesten einen Wirkstoff enthält, der unter den Wirkstoffen gegen Mikroorganismen; entzündungshemmenden oder immunmodulierenden Wirkstoffen; Antagonisten von Neuromediatoren oder Wirkstoffen, die die Aussschüttung von Neuromodulatoren modulieren; Mitteln, die die Differenzierung und/oder Proliferation der Zellen und/oder Pigmentierung modulieren und/oder die Keratinisierung regulieren; Wirkstoffen zur Behandlung und/oder Vorbeugung von Cheilitis; Antihistaminika; und Vernarbungsmitteln ausgewählt ist.

21. Zusammensetzung nach einem der vorhergehenden Ansprüche, die als Lippenstift, Make-up, Wangenrouge, Lidschatten, Mascara, Lippenpflegebasis, Selbstbräunungsmittel und/oder Produkt zum Sonnenschutz vorliegt.

22. Verwendung einer flüchtigen Verbindung in Kombination mit einem Polymer der Formel (I): 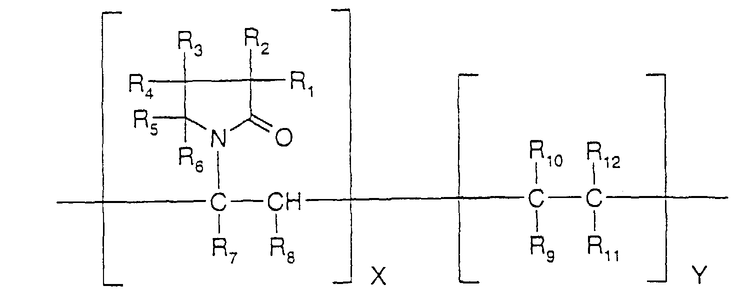 worin die Gruppen R₁ bis R₁₂ unabhängig voneinander eine gesättigte, geradkettige oder verzweigte C₁₀₋₄₀-Alkylgruppe oder ein Wasserstoffatom bedeuten, wobei mindestens eine der Gruppen R₁ bis R₁₂ von Wasserstoff verschieden ist und x nicht 0 ist, in einer kosmetischen Zusammensetzung oder Hygienezusammensetzung oder zur Herstellung einer dermatologischen oder pharmazeutischen Zusammensetzung, um den Transfer und/oder die Migration der Zusammensetzung zu beschränken, zu vermindern und/oder zu unterdrücken.

23. Verwendung von 30 bis 60 Gew.-% einer flüchtigen Verbindung, bezogen auf das Gesamtgewicht der Zusammensetzung, in Kombination mit einem Polymer der Formel (I): 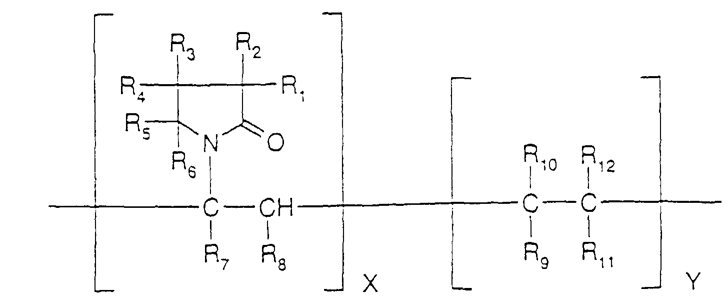 worin die Gruppen R₁ bis R₁₂ unabhängig voneinander eine gesättigte, geradkettige oder verzweigte C₁₀₋₄₀-Alkylgruppe oder ein Wasserstoffatom bedeuten, wobei mindestens eine der Gruppen R₁ bis R₁₂ von Wasserstoff verschieden ist und x nicht 0 ist, in einer kosmetischen Zusammensetzung oder Hygienezusammensetzung oder zur Herstellung einer dermatologischen oder pharmazeutischen Zusammensetzung, um den Transfer und/oder die Migration der Zusammensetzung zu beschränken, zu vermindern und/oder zu unterdrücken.

24. Verwendung nach Anspruch 22, wobei die Zusammensetzung 8 bis 70 Gew.-% und vorzugsweise 30 bis 60 Gew.-% flüchtige Verbindung, bezogen auf das Gesamtgewicht der Zusammensetzung, enthält.

25. Verwendung nach einem der Ansprüche 22 bis 24, wobei mindestens eine der Gruppen, die von Wasserstoff verschieden ist, 14 bis 32 Kohlenstoffatome aufweist.

26. Verwendung nach einem der Ansprüche 22 bis 25, wobei y 0 ist und die Gruppen R₂ bis R₅ Wasserstoff bedeuten und mindestens eine der Gruppen, die von Wasserstoff verschieden sind, 14 bis 32 Kohlenstoffatome aufweist.

27. Verwendung nach einem der Ansprüche 22 bis 26, wobei y nicht 0 ist und die Gruppen R₁ bis R₉ und R₁₁ und R₁₂ Wasserstoff bedeuten, die Gruppe R₁₀ 14 bis 32 Kohlenstoffatome aufweist und das Verhältnis x/y vorzugsweise im Bereich von 1/5 bis 5/1 liegt.

28. Verwendung nach einem der Ansprüche 22 bis 27, wobei das Polymer unter dem Copolymer PVP/Hexadecen, dem Copolymer PVP/Eicosen, dem Polymer Triacontanyl-PVP und deren Gemischen ausgewählt ist.

29. Verwendung nach einem der Ansprüche 22 bis 28, wobei das Polymer in der Zusammensetzung in Mengenanteilen von 0,5 bis 40 Gew.-% und vorzugsweise 1 bis 20 Gew.-% vorliegt.

30. Verwendung nach einem der Ansprüche 22 bis 29, wobei die flüchtige Verbindung unter den Kohlenwasserstoffölen und/oder den cyclischen oder linearen Siliconölen oder deren Gemischen ausgewählt ist.

31. Verwendung nach einem der Ansprüche 22 bis 30, wobei die Zusammensetzung mindestens eine nichtflüchtige Fettsubstanz enthält, die unter den Ölen, Gummis, pastösen Fettsubstanzen und/oder Wachsen, die pflanzlich, mineralisch, tierisch, synthetisch, siliconhaltig und/oder fluoriert sein können, ausgewählt ist.

32. Verwendung nach einem der Ansprüche 22 bis 31, wobei die Zusammensetzung 0 bis 45 Gew.-% Kohlenwasserstofföl und vorzugsweise 20 bis 40 Gew.-% enthält.

33. Verwendung nach einem der Ansprüche 22 bis 32, wobei die Zusammensetzung ferner eine hydrophile Komponente enthält, die unter mindestens einem mehrwertigen Alkohol und/oder einer dickflüssigen oder sogar gelierten wäßrigen Phase ausgewählt ist.

34. Verwendung nach einem der Ansprüche 22 bis 33 in Lippenstiften, Make-up, Wangenrouge, Lidschatten, Mascara, Lippenpflegeprodukten, Selbstbräunungsmitteln und/oder Produkten zum Sonnenschutz.

## Claims

1. Cosmetic, dermatological, hygiene and/or pharmaceutical transfer-resistant stick comprising from 30 to 60% by weight of a volatile compound, relative to the total weight of the composition, at least one wax and a polymer of formula (I): 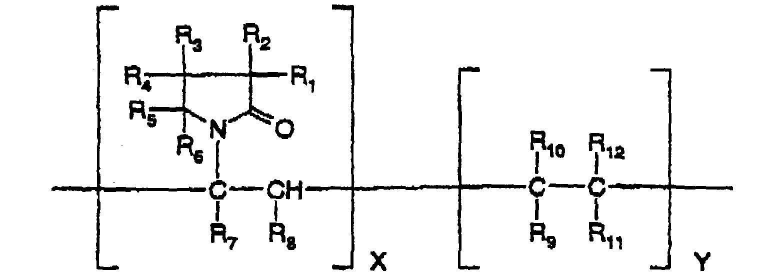 in which the radicals R1 to R12 represent, independently of each another, a saturated alkyl radical containing a straight or branched C10 to C40 chain or a hydrogen atom, at least one of the said radicals R1 to R12 being other than a hydrogen atom; x being non-zero.

2. Composition according to Claim 1, in which at least one of the radicals R1 to R12 represents an alkyl radical containing 14 to 32 carbon atoms, preferably 28 to 32 carbon atoms.

3. Composition according to either of the preceding claims, in which the polymer has a weight-average molecular mass of between 5000 and 30,000, in particular between 6000 and 20,000.

4. Composition according to one of the preceding claims, in which y is equal to 0 and the radicals R2 to R5 represent hydrogen.

5. Composition according to Claim 4, in which at least one of the radicals other than hydrogen contains from 14 to 32 carbon atoms.

6. Composition according to one of Claims 1-3, in which y is non-zero and the radicals R1 to R9 and R11 and R12 represent hydrogen.

7. Composition according to Claim 6, in which R10 contains from 14 to 32 carbon atoms, and the ratio x/y is preferably between 1/5 and 5/1.

8. Composition according to one of the preceding claims, in which the polymer is chosen from the group consisting of the PVP/hexadecene copolymer, the PVP/eicosene copolymer and the triacontanyl-PVP polymer, and mixtures thereof.

9. Composition according to one of the preceding claims, in which the polymer is present in the composition in a proportion of from 0.5 to 40% by weight, preferably 1 to 20% by weight.

10. Composition according to one of the preceding claims, in which the volatile compound is chosen from linear or cyclic hydrocarbon-based oils and/or silicone oils, alone or as a mixture.

11. Composition according to one of the preceding claims, in which the volatile compound is chosen from cyclic volatile silicones containing from 3 to 8 silicon atoms, preferably from 4 to 6 silicon atoms; cyclocopolymers such as dimethylsiloxane/methylalkylsiloxane; linear volatile silicones containing from 2 to 9 silicon atoms; alkyltrisiloxanes; isoparaffins; mixtures thereof.

12. Composition according to one of the preceding claims, also comprising at least one non-volatile fatty substance chosen from oils, gums, pasty fatty substances and/or waxes of plant, mineral, animal, synthetic, silicone and/or fluoro origin.

13. Composition according to one of the preceding claims, in which the wax is chosen from microcrystalline waxes, paraffin, petrolatum, petroleum jelly, ozokerite, montan wax; beeswax, lanolin and its derivatives; candelilla wax, ouricury wax, carnauba wax, Japan wax, cocoa butter, cork fibre wax or sugar cane wax; hydrogenated oils that are solid at 25°C, ozokerites, fatty esters and glycerides that are solid at 25°C; polyethylene waxes and the waxes obtained by Fischer-Tropsch synthesis; silicone waxes; fluoro waxes, mixtures thereof.

14. Composition according to one of the preceding claims, also comprising at least one phenylsilicone oil corresponding to the following formula: 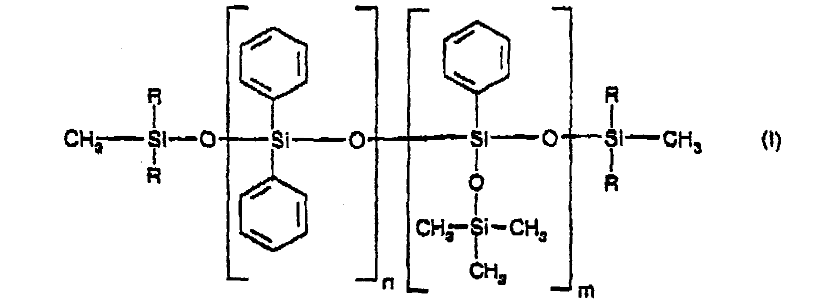 in which
. R is a C1-C30 alkyl radical, an aryl radical or an aralkyl radical,
. n is an integer between 0 and 100,
. m is an integer between 0 and 100, with the proviso that the sum m+n is between 1 and 100.

15. Composition according to one of the preceding claims, also comprising at least one hydrocarbon-based oil chosen from liquid paraffin, liquid petroleum jelly, perhydrosqualine, groundnut oil, sweet almond oil, macadamia oil, grape seed oil, rapeseed oil, coconut oil, beauty-leaf oil, palm oil, castor oil, avocado oil, apricot oil, sesame oil, jojoba oil, olive oil or cereal germ oil; fatty acid esters; alcohols; acetyl glycerides; alkyl or polyalkyl octanoates, decanoates or ricinoleates; fatty acid triglycerides; glycerides, mixtures thereof.

16. Composition according to Claim 15, in which the content of hydrocarbon-based oil is between 0 and 45% by weight, preferably 20 to 40% by weight.

17. Composition according to one of the preceding claims, also comprising a hydrophilic part chosen from at least one polyhydric alcohol and/or a thickened, or even gelled, aqueous phase.

18. Composition according to Claim 17, in which the hydrophilic part represents 0 to 40% by weight of the composition, preferably 8 to 20% by weight.

19. Composition according to one of the preceding claims, also comprising a particulate phase comprising pigments and/or nacres and/or fillers.

20. Composition according to one of the preceding claims, also comprising at least one active agent chosen from agents that are active against microorganisms; agents with anti-inflammatory or immunomodulatory activity; neuromediator antagonists or agents which modify the release of neuromediators; agents which modify cell differentiation and/or proliferation and/or pigmentation and/or which regulate keratinization; active agents in the treatment and/or prevention of cheilitis; antihistamines; cicatrizing agents.

21. Composition according to one of the preceding claims, which is in the form of a lipstick, a foundation, a face powder, an eyeshadow, a mascara, a lipcare product, a self-tanning product and/or an antisun product.

22. Use, in a cosmetic or hygiene composition or for the manufacture of a dermatological or pharmaceutical composition, of the combination of a volatile compound and a polymer of formula I: 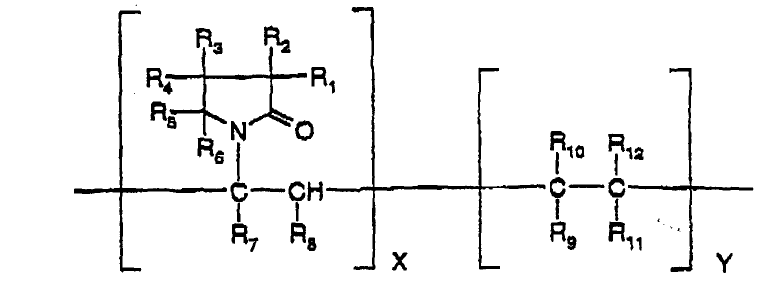 in which the radicals R1 to R12 represent, independently of each other, a saturated alkyl radical containing a straight or branched C10 to C40 chain or a hydrogen atom, at least one of the said radicals R1 to R12 being other than a hydrogen atom; x being non-zero, as an agent for limiting, decreasing and/or preventing the transfer and/or migration of the said composition.

23. Use, in a cosmetic or hygiene composition or for the manufacture of a dermatological or pharmaceutical composition, of from 30 to 60% by weight, relative to the total weight of the composition, of a volatile compound and of a polymer of formula (I) 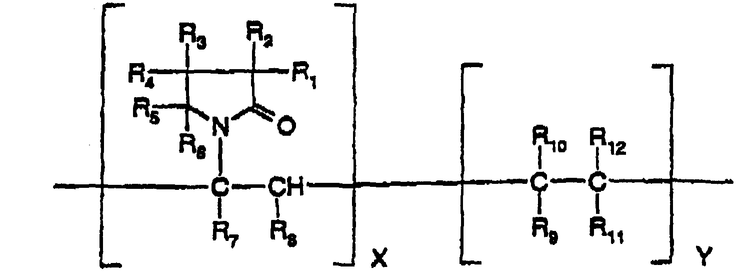 in which the radicals R1 to R12 represent, independently of each other, a saturated alkyl radical containing a straight or branched C10 to C40 chain or a hydrogen atom, at least one of the said radicals R1 to R12 being other than a hydrogen atom; x being non-zero, in order to limit, decrease and/or prevent the transfer and/or migration of the said composition.

24. Use according to Claim 22, in which the composition comprises 8 to 70% by weight, preferably 30 to 60% by weight, of volatile compound relative to the total weight of the composition.

25. Use according to one of Claims 22 to 24, in which at least one of the radicals other than hydrogen comprises from 14 to 32 carbon atoms, preferably 28 to 32 carbon atoms.

26. Use according to one of Claims 22 to 25, in which y is equal to 0, the radicals R2 to R5 represent hydrogen and at least one of the radicals other than hydrogen comprises from 14 to 32 carbon atoms.

27. Use according to one of Claims 22 to 26, in which y is non-zero, the radicals R1 to R9 and R11 and R12 represent hydrogen, the radical R10 contains from 14 to 32 carbon atoms and the ratio x/y is preferably between 1/5 and 5/1.

28. Use according to one of Claims 22 to 27, in which the polymer is chosen from the group consisting of the PVP/hexadecene copolymer, the PVP/eicosene copolymer and the triacontanyl-PVP polymer, and mixtures thereof.

29. Use according to one of Claims 22 to 28, in which the polymer is present in the composition in a proportion of from 0.5 to 40% by weight, preferably 1 to 20% by weight.

30. Use according to one of Claims 22 to 29, in which the volatile compound is chosen from linear or cyclic hydrocarbon-based oils and/or silicone oils, alone or as a mixture.

31. Use according to one of Claims 22 to 30, in which the composition also comprises at least one non-volatile fatty substance chosen from oils, gums, pasty fatty substances and/or waxes of plant, mineral, animal, synthetic, silicone and/or fluoro origin.

32. Use according to one of Claims 22 to 31, in which the composition comprises 0 to 45% by weight of hydrocarbon-based oil, preferably 20 to 40% by weight.

33. Use according to one of Claims 22 to 32, in which the composition also comprises a hydrophilic part chosen from at least one polyhydric alcohol and/or a thickened, or even gelled, aqueous phase.

34. Use according to one of Claims 22 to 33, in a lipstick, a foundation, a face powder, an eyeshadow, a mascara, an eyeliner, a lipcare product, a self-tanning product and/or an antisun product.
